# EUROPEAN PATENT APPLICATION

(11) **EP 2 474 622 A1**
(43) Date of publication of application: **11.07.2012**
(21) Application number: 10813383.6
(22) Date of filing: 03.09.2010
(51) Int. Cl.: C12Q 1/68

(54) **METHOD FOR THE DIAGNOSIS AND/OR PROGNOSIS OF ACUTE RENAL DAMAGE**

(30) Priority: 04.09.2009 ES 200901825
(71) Applicant: Fundación Para La Investigación Biomédica Del Hospital Universitario Ramón Y Cajal, 28034 Madrid (ES)
(72) Inventor: GARCIA BERMEJO, Maria Laura, E-28034 Madrid (ES); AGUADO FRAILE, Elia, E-28034 Madrid (ES); LIAÑO GARCIA, Fernando, E-28034 Madrid (ES); SAENZ MORALES, David, E-28034 Madrid (ES)
(74) Representative: Temino Ceniceros, Ignacio
(86) International application number: PCT/ES2010/070579
(87) International publication number: WO 2011/027019

(57) **Abstract**

The invention relates to a method for the diagnosis and/or prognosis of acute renal damage by analysing the level of expression of at least one microRNA selected from miR-127, miR-126, miR-210 and miR-101.

## Description

### Field of the invention

The present invention generally falls within the field of biomedicine and in particular refers to a method for the diagnosis and/or prognosis of acute renal damage.

### Background of the invention

In kidney transplant, Acute Tubular Necrosis (ATN) is the main cause of the delay in the post-transplant graft function. In addition, the ATN contributes to an increased incidence of acute rejection, the development of chronic rejection and graft survival decreased (Pannu et al., 2008). The increase in the demand for organs in recent years involves the use of organs from sub-optimal donors, including asystolic and aged donors, which significantly increases the percentage of development of post transplant ATN, the morbidity of the graft and the delay in its functional recovery. This pushes up the total economic cost of a kidney transplant to public health. Note that the latest statistics of the National Transplant Organization (NTO) indicate the realization of about 2,200 kidney transplants/year in Spain and more than 4,000 patients still on waiting list (Dominguez-Gil and Pascual. 2008). On the other hand, the ATN is the commonest morphologic manifestation of Acute Renal Failure (ARF), including ischemic origin (Kellum et al., 2008). The ARF represents one of the most serious problems within the kidney diseases in the developed world due to its high mortality, around 50%. Around 30% of all events of ARF occur in patients admitted to ICUs, as a result of multiple organ failure. In the latter context, the mortality rises to 80% (Chertow et al., 2005). The development of ARF is also one of the most common complications after cardiac intervention, of which around 30,000/year are carried out in Spain, and more than one 1% of them in our Hospital. Practically all of the operated patients develop certain degree of ARF (Yates and Stafford-Schmit, 2006). From the severity of this post-operative ARF depends the long-term progress of the patients, resulting in a mortality approaching 60% in those cases requiring dialysis after the cardiac surgery (Takar et al., 2005; Candela-Toha et al., 2008). Both kidney transplantation and cardiac surgery are two "quasi" experimental study situations for the human ATN, since it is known the moment and duration of the ischemic stimulus and they can also be monitored. All these statistics of morbi-mortality have not changed significantly in recent decades and so far, an effective therapy for prevention and/or reduction of the ATN in all these situations does not exist. To this has greatly contributed the lack of markers of kidney damage more accurate than the determination of serum creatinine and urea, used until now. These classic markers do not directly reflect the cell damage or in that compartment of the kidney tissue (tubule or endothelium) is being produced the same; they are only indicative parameters of altered renal function as a result of the damage (Vaidya et al., 2008). In fact, it is possible that patients with a subclinical renal damage are not identified as such because a significant alteration in serum creatinine and urea levels has not been produced. Thus, in recent years numerous studies are being developed which are trying to identify and validate new markers of the ARF like NGAL, IL 18, KIM, Cystatin C, VEGF or CXCL 10, which seem to work as good markers in infantile populations without significant added pathologies but not in adult population (Vaidya et al., 2008).

Renal ischemia, hypovolemia and toxics are the most frequent causes of development of ATN. The reduction in blood flow and as a result the tissue hypoxia result in damage at the level of the proximal tubular epithelium, cause a rapid decrease in the glomerular filtrate, alter vascular permeability and provoke an inflammatory response that amplifies the tissue damage (Thurman et al., 2007). The degree and extent of the ischemic damage are dependent on the severity and duration of ischemia. In sublethal ischemia, it can be seen the detachment of the proximal epithelium cells, many of them viable, to the tubular lumen. In prolonged ischemia, the persistent tissue hypoxia and the inflammatory response, *inter alia,* increase the epithelial and vascular damage, with cell death in the cortical-medullar area of the kidney. On the other hand, the vascular compartment is also damaged after ischemia. In fact, the endothelial damage significantly contributes to the acute renal damage and also to the maintenance of the same in the time. Early alterations in the peritubular flow during ischemia and early reperfusion are associated with loss of endothelial morphology and function contributing to the loss of function of barrier, inflammation and procoagulant activity. At medium-long term, it has described loss of microvascular density that favors the progression of chronic kidney damage as a direct result of the initial ischemia (Basile 2007). To resolve the ATN, mechanisms that facilitate tissue repair are started: Cell division and differentiation from epithelial tubular cells not damaged. In recent years, several studies have demonstrated that to the repair of the tubular damage after ischemia can contribute not only the epithelial not damaged cells themselves that are de-differentiated and proliferate, but kidney pluripotent cells and even extrarenal pluripotent cells as those from bone marrow (Lin 2008). However, the contribution of stem cells to repair the ischemic damage is questioned, although it is accepted that to it contribute fundamentally proximal tubular cells not damaged and parenchyma revascularization. In this latter process, it has been proposed that endothelial parents mobilized after ischemia would also participate (Becherucci et al., 2009).

The miRNAs are small in size (22-25 nucleotides) endogenously encoded RNAs able to recognize messenger RNAs and thus negatively regulate the expression of proteins, within induced silencing complexes (RISC) by total or partial complementarity with its target mRNA (Chang and Mendell, 2007). In humans more than 700 have already cloned and bioinformatic predictions indicate that all of them can control the expression of more than 30% of the total proteins (Filipowicz et al., 2008). The majority are transcribed by RNA Pol II from individual genes or from transcribed polycistronics for several of them at the same time. They are generated as longer pre-miRs processed in the core by a Ribonuclease III (Drosha), go to cytoplasm via Exportin-5- and Ran-GTP-dependent mechanisms and there they are finally processed by another Ribonuclease III (Dicer) to its mature form (Rana 2007). Its function is essential in a wide variety of processes including the embryonic development, response to stress or strict regulation of physiological processes and therefore, the maintenance of homeostasis in organisms. It is important to highlight that miRNAs expression profile is specific to cell type and can change depending on the stimulus, so that the particular cell context of a same miRNA will determine its function in a specific cell type (Bartel 2009). For this reason, the deregulation of certain miRNAs has been pointed out among the mechanisms responsible for the development of pathologies such as cancer (Bartels and Tsongalis, 2009), autoimmunity (Sonkoly and Pivarcsi 2008), diabetes (Zhou et al., 2008) or vascular pathologies (Urbich et al., 2008) and they are making up as precise biomarkers of the progress of many of them. Very recently it has been demonstrating that in addition the miRNAs are key regulators in the cellular response quickly and accurately before any type of stimulus including the lack of nutrients or hypoxia (Ivan et al., 2008). On the other hand, it has also been demonstrated that these miRNAs along with mRNAs can be secreted or exchanged by the cells in the form of micro-particles (microvesicles of platelets; exosomes in tumor cells; ectosomes of neutrophils (Valadi et al., 2007). They could thus be detected in body fluids such as blood, urine or pleural fluid. In fact, it is estimated that the peripheral blood of healthy individuals can contain a concentration between 5-50 mg/ml of micro-particles, which would increase in the event of patients with various pathologies (Hunter et al., 2008). This would allow performing a very reliable monitoring of the progress of these pathologies using samples obtained by methods minimally invasive (blood extraction and urine collection (Gilad 2008). In urine, the detected miRNAs, among which is the miR-127, have shown great stability, yet under very aggressive conditions (Melkonyan et al., 2008). Given that the deregulation of miRNAs can cause various pathologies, they are beginning to be regarded as new targets of therapeutic action. In fact, it has been developed tools for modulating its expression: pre-miRs for their over-expression and antagomiRs (anti-miRs) for their inhibition, with very hopeful results in various experimental models *in vitro* and *in vivo* (Krutzfeld et al., 2006; Care et al., 2007; Van Rooij et al., 2008), although its validity still has to be determined as therapeutic strategy in humans.

Regarding to the role of miRNAs in response to ischemia, it has been determined its expression in focal cerebral ischemia in rat, being established association between the expression of miR-145 and brain damage (Dharap et al., 2009). In cardiac ischemia in humans the miR-100 and the miR-133 seem to participate in the mechanism of cardiac damage (Sucharov C, et al., 2008). In hepatic ischemia also in humans, miR-223 has been established as a mediator of damage (Yu et al., 2008). On the contrary, miR-126 and miR-210 have been described as key promoters of angiogenesis, neovascularization and tissue repair in response to various stimuli, including hypoxia (Suarez and Sessa, 2009; Fasanaro et al., 2008; van Solingen et al., 2008). So far, miRNAs modulated in renal R/I have not described in literature, but certainly it has been began to speculate with their potential as biomarkers in renal pathologies, including those involving disturbances in the regulation of blood pressure (Liang M et al., 2009). In another context, some miRNAs associated with immune rejection in kidney transplantation have been identified (Sui et al., 2008).

All stated above justifies the need to identify and validate new biomarkers of evolution of kidney damage more accurate and indicative of what tissue compartment and in which extent is damaging and/or recovering, whose determination is further fast, easy and without the need for biopsy to the patient.

### Description of the invention

Thus, in a first aspect, the present invention provides a method for the diagnosis and/or prognosis of acute renal damage comprising analyzing a sample of a patient, to determinate the level of expression of at least un microRNA selected from miR-127, miR-126, miR-210 and miR-101 and compare said level of expression with a control value, where the alteration of said level is indicative of renal damage.

In one aspect more particularly of the present invention, the sample of the patient to be analyzed is blood. In another aspect in particular of the present invention, the sample is serum. In another aspect of the present invention, the sample is urine.

In one aspect more particularly, the diagnosis and/or prognosis of acute renal damage is carried out by determining of level of expression of the miR-127 alone or in combination with at least un microRNA selected from miR-126, miR-210 and miR-101.

In one aspect more particularly, the decrease of the level of expression of serum miR-127 with regard to the control value is indicative of acute renal damage.

In one aspect more particularly, the increase of the level of expression of urinary miR-127 with regard to the control value is indicative of acute renal damage.

In one aspect more particularly, the diagnosis and/or prognosis of acute renal damage is carried out by determining of level of expression of the miR-126 alone or in combination with at least un microRNA selected from miR-127, miR-210 and miR-101.

In one aspect more particularly, the decrease of the level of expression of serum miR-126 with regard to the control value is indicative of acute renal damage.

In one aspect more particularly, the diagnosis and/or prognosis of acute renal damage is carried out by determining of level of expression of the miR-210 alone or in combination with at least un microRNA selected from miR-127, miR-126 and miR-101.

In one aspect more particularly, the increase of the level of expression of serum miR-210 with regard to the control value is indicative of acute renal damage.

In one aspect more particularly, the decrease of level of expression of urinary miR-210 with regard to the control value is indicative of acute renal damage.

In one aspect more particularly, the diagnosis and/or prognosis of acute renal damage is carried out by determining of level of expression of the miR-101 alone or in combination with at least un microRNA selected from miR-127, miR-126 and miR-210.

In one aspect more particularly, the increase of level of expression of serum miR-101 with regard to the control value is indicative of acute renal damage.

In the present invention by acute renal damage refers to kidney damage with ischemic etiology, either primary or secondary, as it is the case of kidney damage due to toxics or by radiocontrast means, and in any case, excluding chronic kidney damage.

In one aspect more particularly of the present invention, the expression of microRNA is determined by PCR. In one aspect more particularly, the expression of microRNA is determined by quantitative PCR. In one aspect more particularly, the expression of microRNA is determined by multiplex PCR.

In another aspect more particularly of the present invention, the expression of microRNA is determined by total ARN microarrays.

In a second aspect, the present invention refers to a kit for the diagnosis and/or prognosis of acute renal damage comprising the probes and primers needed for carrying out the method of the present invention.

In one aspect more particularly of the present invention, the kit comprises the probes and primers needed for determining the level of expression of at least a microRNA selected from miR-127, miR-126, miR-210 and miR-101.

In another aspect more particularly of the present invention, the kit comprises a RNA microarray.

### Description of the figures

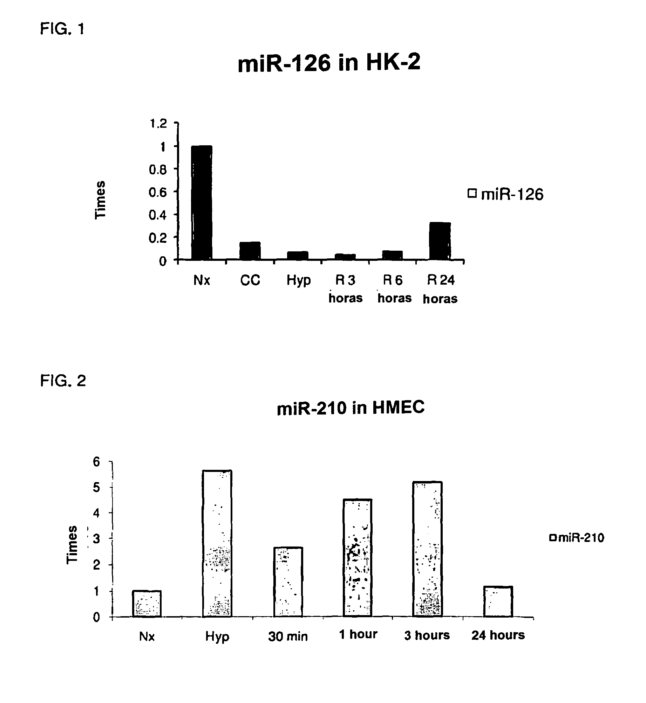

**Figure 1** shows the expression of miR-126 in HK-2 human proximal tubular cells subjected to the Hypoxia/Reoxygenation Protocol. NX: cells under normal conditions with regard to oxygen availability (normoxia, 21 %) and nutrients availability (complete medium with 10% FBS) CC: control cells that have suffered nutrients restrictions (they are in free-nutrient medium). Hyp: cells that have suffered nutrient and oxygen restrictions during 6 h (oxygen 1% in free-nutrient medium); R-3, R-6, R-24 h: cells that after 6 h of hypoxia are again under normal conditions of oxygen and nutrient availability.
**Figure 2** shows the expression of miR-210 in HK-2 human proximal tubular cells subjected to the Hypoxia/Reoxygenation Protocol. NX: cells under normal conditions with regard to oxygen availability (normoxia, 21 %) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-3, R-6, R-24 h: cells that are again under normal conditions of oxygen and nutrient availability.

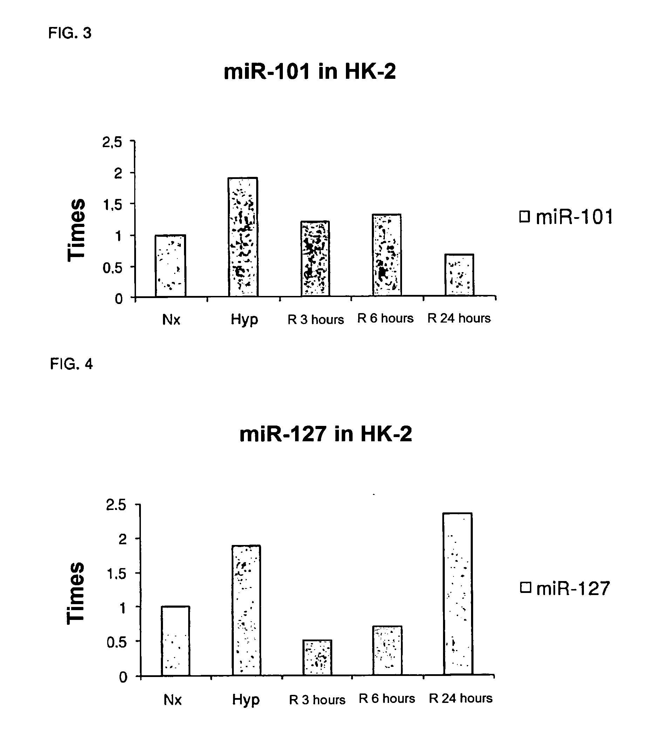

**Figure 3** shows the expression of miR-101 in HK-2 human proximal tubular cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-3, R-6, R-24 h: cells that are again under normal conditions of oxygen and nutrient availability.
**Figure 4** shows the expression of miR-127 in HK-2 human proximal tubular cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-3, R-6, R-24 h: cells that are again under normal conditions of oxygen and nutrient availability.

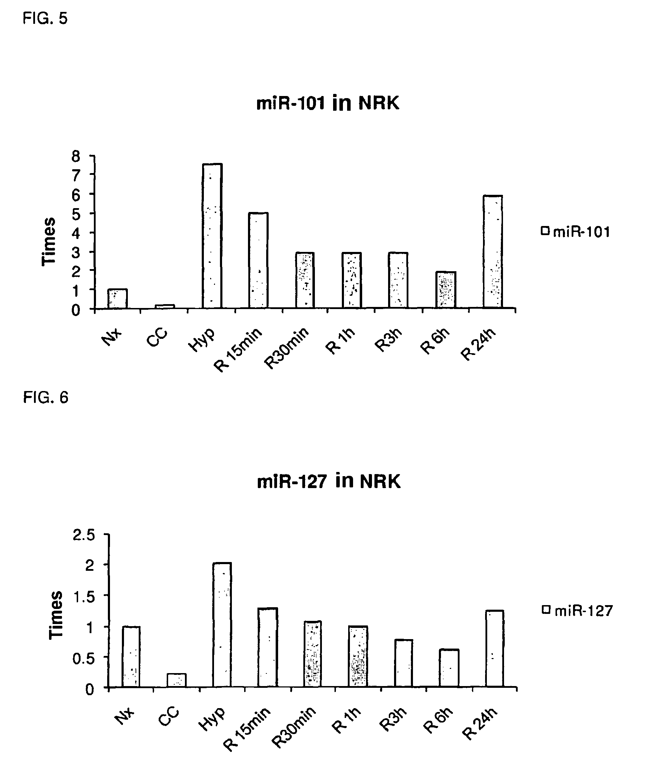

**Figure 5** shows the expression of miR-101 in NRK-52E rat proximal tubular cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-15 min, R-30 min, R-1 h, R-3 h, R-6 h, R-24 h: cells that are again under normal conditions of oxygen and nutrient availability.
**Figure 6** shows the expression of miR-127 in NRK-52E rat proximal tubular cells subjected to the Hypoxia/Reoxygenation Protocol. NX: cells under normal conditions with regard to oxygen availability (normoxia, 21 %) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-15 min, R-30 min, R-1 h, R-3 h, R-6 h, R-24 h: cells that are again under normal conditions of oxygen and nutrient availability.

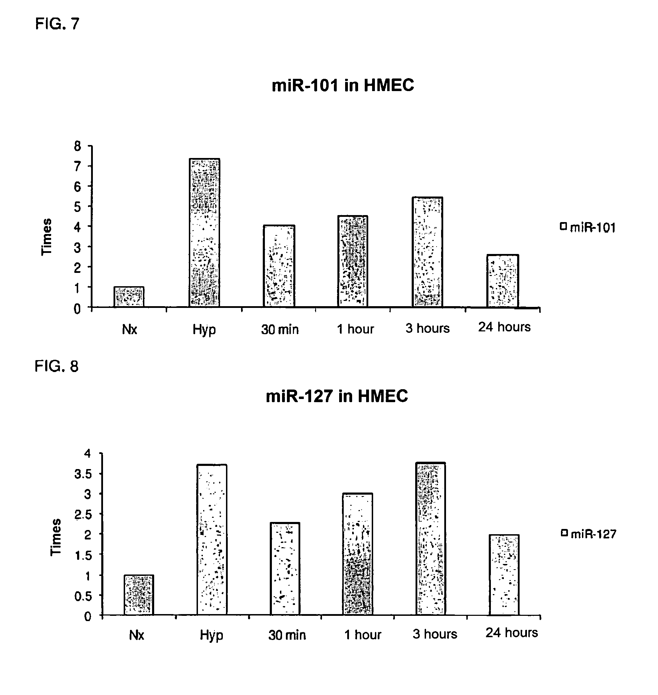

**Figure 7** shows the expression of miR-101 in HMEC human endothelial cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-15 min, R-30 min, R-1 h, R-3 h, R-24 h: cells that are again under normal conditions of oxygen and nutrient availability.
**Figure 8** shows the expression of miR-127 in HMEC human endothelial cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-15 min, R-30 min, R-1 h, R-3 h, R-24 h: cells that are again under normal conditions of oxygen and nutrient availability.

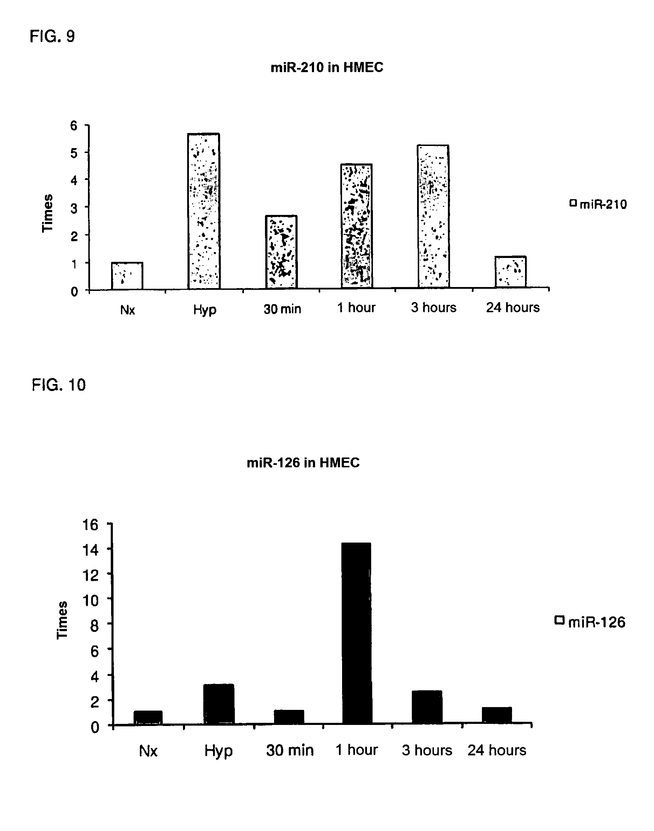

**Figure 9** shows the expression of miR-210 in HMEC human endothelial cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-15 min, R-30 min, R-1 h, R-3 h, R-24 h: cells that are again under normal conditions of oxygen and nutrient availability.
**Figure 10** shows the expression of miR- 126 in HMEC human endothelial cells subjected to the Hypoxia/Reoxygenation Protocol. NX: cells under normal conditions with regard to oxygen availability (normoxia, 21%) and nutrients availability (complete medium with 10% FBS) CC: control cells that have suffered nutrients restrictions (they are in free-nutrient medium). Hyp: cells that have suffered nutrient and oxygen restrictions (oxygen 1% of free-nutrient medium); R-15 min, R-30 min, R-1 h, R-3 h, R-24 h: cells that are again under normal conditions of oxygen and nutrient availability.

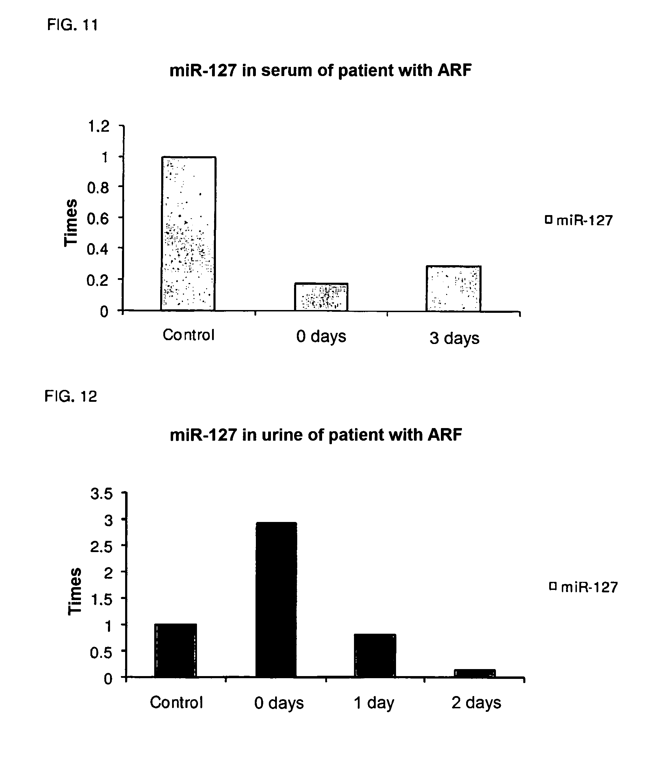

**Figure 11** shows the expression of serum miR-127 of patients diagnosed with Acute Renal Failure (ARF) of ischemic etiology. Control: expression of miRNA in healthy control equaled to 1. Expression data in the patient are relativized to this data. 0 h, and 3 days: times in which has been taken sample of the patient, on admittance by ARF (0 h) and later in its progress (3 days).
**Figure 12** shows the expression of urinary miR-127 of patients diagnosed with Acute Renal Failure (ARF) of ischemic etiology. Control: expression of miRNA in healthy control equaled to 1. Expression data in the patient are relativized to this data. 0 h, and 3 days: times in which has been taken sample of the patient, on admittance by ARF (0 h) and later in its progress (3 days).

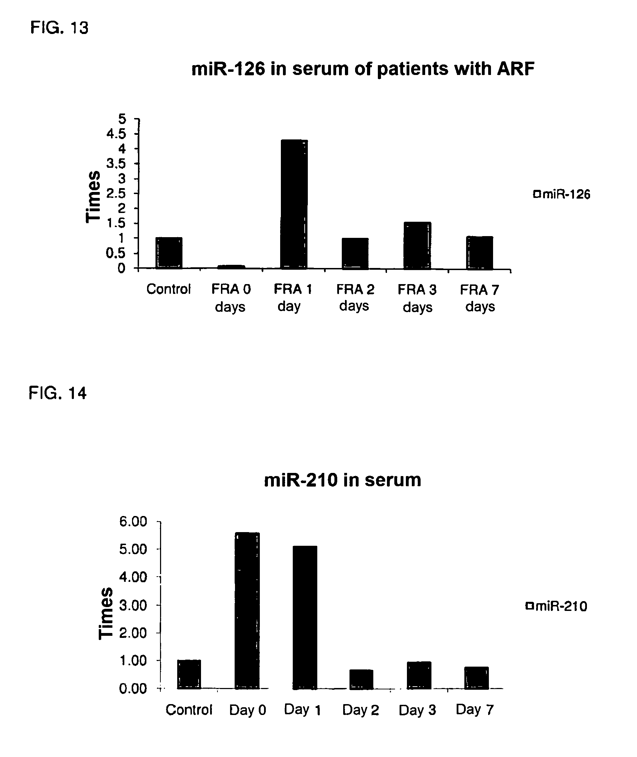

**Figure 13** shows the expression of serum miR-126 of patients diagnosed with Acute Renal Failure (ARF) of ischemic etiology. Control: expression of miRNA in healthy control equaled to 1. Expression data in the patient are relativized to this data. 0 h, and 3 days: times in which has been taken sample of the patient, on admittance by ARF (0 h) and later in its progress (3 days).
**Figure 14** shows the expression of serum miR-210 of patients diagnosed with Acute Renal Failure (ARF) of ischemic etiology. Control: expression of miRNA in healthy control equaled to 1. Expression data in the patient are relativized to this data. Day 0, Day 1, Day 2, Day 3, Day 7: times in which has been taken sample of the patient, on admittance by ARF (0 h) and later in its progress.

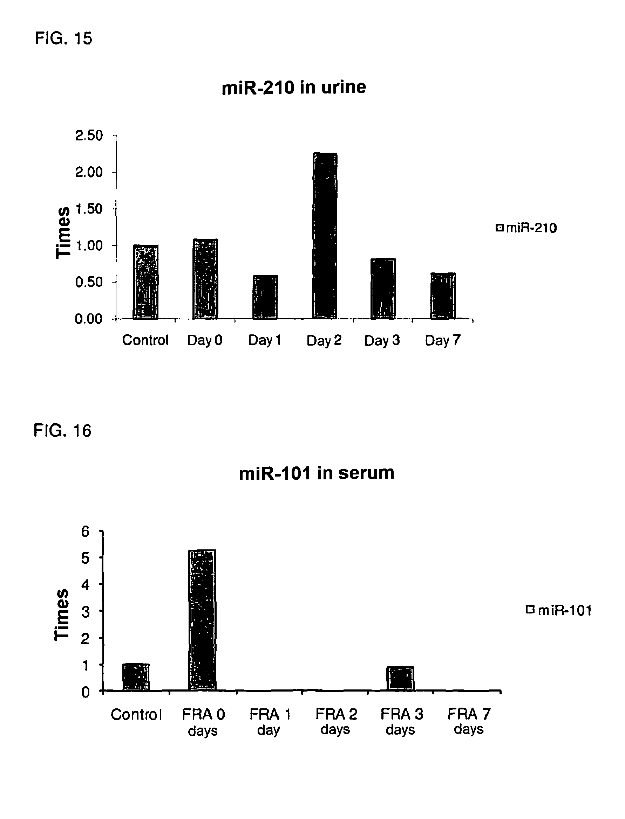

**Figure 15** shows the expression of urinary miR-210 of patients diagnosed with Acute Renal Failure (ARF) of ischemic etiology. Control: expression of miRNA in healthy control equaled to 1. Expression data in the patient are relativized to this data. Day 0, Day 1, Day 2, Day 3, Day 7: times in which has been taken a sample of the patient, on admittance by ARF (0 h) and later in its progress.
**Figure 16** shows the expression of serum miR-101 of patients diagnosed with Acute Renal Failure (ARF) of ischemic etiology. Control: expression of miRNA in healthy control equaled to 1. Expression data in the patient are relativized to this data. 0 days, 1 day, 2 days, 3 days, 7 days: times in which has been taken a sample of the patient, on admittance by ARF (0 d) and later in its progress.

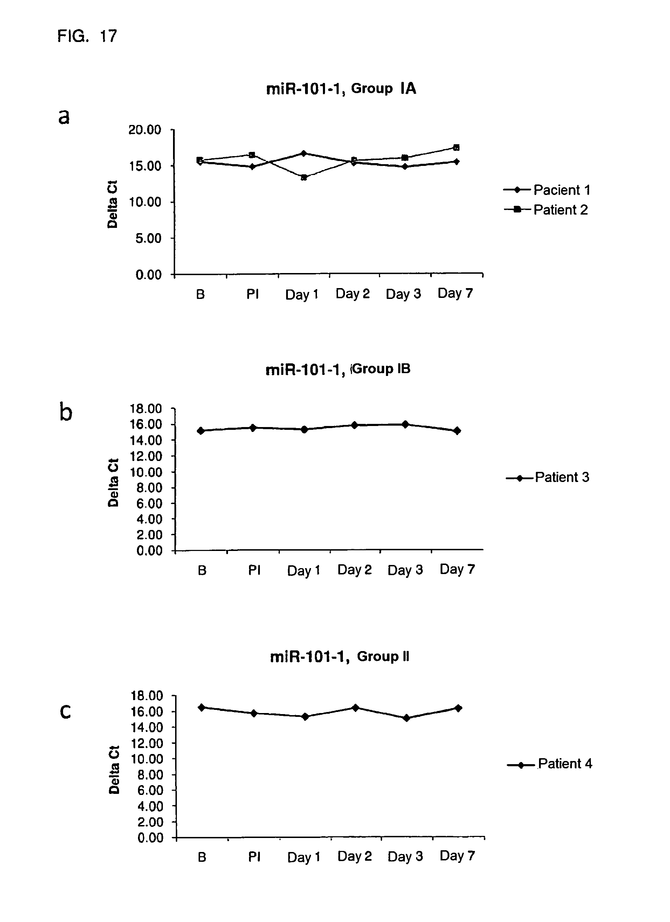

**Figure 17** shows the expression of miR-101 in serum samples a: in patients belonging to group IA, b: in patients belonging to group IB, y c: in patients belonging to group II.

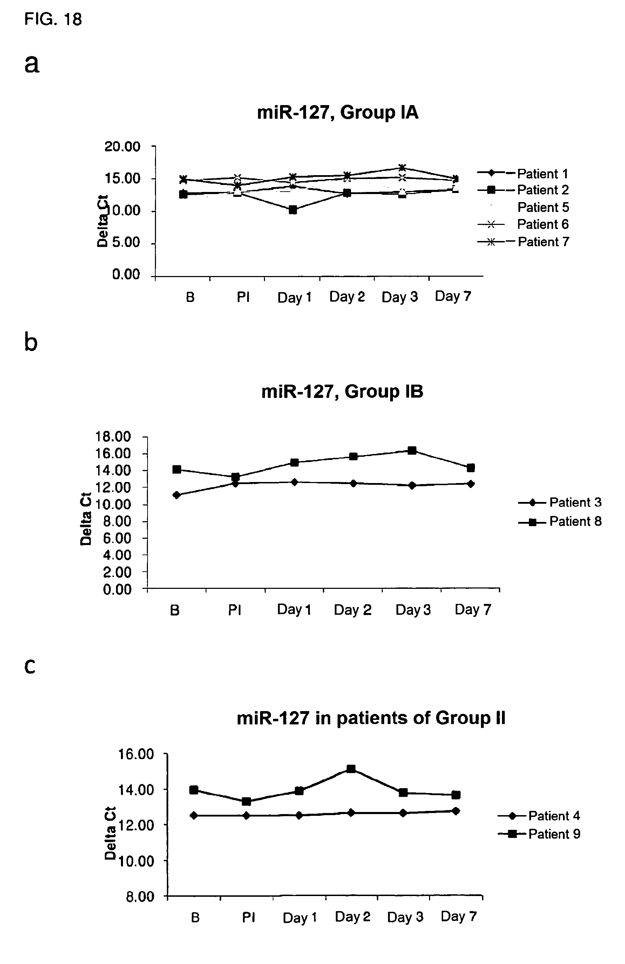

**Figure 18** shows the expression of miR-127 in serum samples a: in patients belonging to group IA, b: in patients belonging to group IB, y c: in patients belonging to group II.

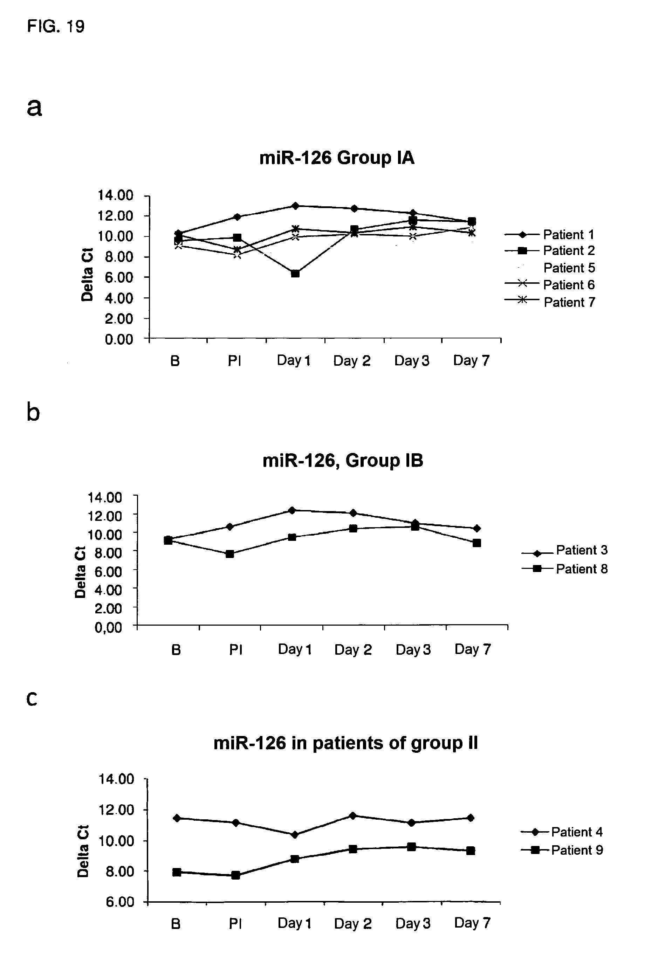

**Figure 19** shows the expression of miR-126 in serum samples a: in patients belonging to group IA, b: in patients belonging to group IB, y c: in patients belonging to group II.

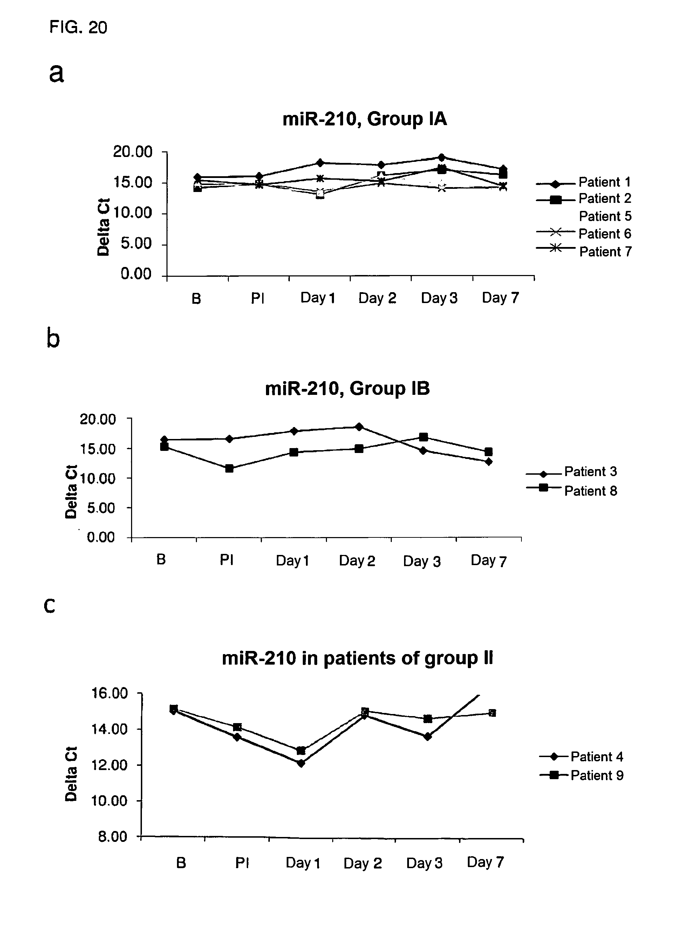

**Figure 20** shows the expression of miR-210 in serum samples a: in patients belonging to group IA, b: in patients belonging to group IB, y c: in patients belonging to group II.

### Detailed description of the invention

There were identified by the use of arrays that the microRNAs: miR-127, miR-126, miR-210 and miR-101 in a HR model which disguising R/I, expressed in a differential way so that each of these microRNAs, alone or in combination serve as biomarkers of kidney damage.

### EXAMPLE 1: Expression of miRNAs in line cells in cell lines subjected to hipoxia/reoxygenation.

There were proceeded to the culture of the following cells (HK2: human proximal tubular cells, NRK-52E: rat proximal tubular cells and HMEC: human microvascular endothelial cells) in appropriate means containing specific serum, antibiotics and growth factors. They kept at 37 °C, under moist atmosphere and with CO₂ 5%.

Cell lines described above were subjected to the hipoxia/reoxygenation protocol. That is, cell lines are subjected to changes in oxygen tensions and in the availability of nutrients. For that purpose, two different incubators were used: hypoxia in a sealed incubator at 37 °C, perfused with a mixture of CO₂ 5%, O₂ 1%, N₂ 94%; reoxygenation in a standard incubator at 37°C, with CO₂ 5%. The cells were grown to confluence and deprived of serum 24 hours before the hypoxia. During hypoxia, they kept in free-serum minimal medium (HBSS), with low concentration of glucose or derivatives. During reoxygenation, it was used a complete medium (Saenz-Morales et al., 2006). The hypoxia time for all samples was 6 h, and reoxygenation times were variable (15 min-72 h). All of the experiments *in vitro* were repeated at least 3 times.

Next the expression of distinct microRNAs in cell lines by PCR was determined, for this purpose and after extraction of total RNA from samples of fluids (serum or urine) and titrates it, 50 nanograms of each one of them was used for the reaction of retrotranscription (RT) in 15 microliters. Special commercial primers were used for this step (stem loop primers). These primers were specific to each miRNA. After the RT, it was undertaken to the amplification reaction in a quantitative manner (qPCR). In this reaction that was carried out in a 10 microliters total volume, was used 1 microliter of the RT total reaction and specific primers for each miRNA and furthermore Taqman probe with fluorescence catchers. All reagents, both primers and reaction mixtures with enzymes and nucleotides for RT and PCR were utilized from Applied Biosystems. The results were as follows:

As shown in Figure 1, the levels of expression of miR-126, were heavily dependent on the availability of nutrients and oxygen, since the restriction of both decreased very significantly its expression with respect to the normality condition. The expression of miRNA was recovering in time of reoxygenation, where cells returned to dispose of oxygen and nutrients. Hence the decrease in the expression of miR-126 indicated lack of nutrients and oxygen in the proximal tubular cells being indicative of renal ischemia. On the other hand, and given that at 3 h of reoxygenation was recorded an important damage to the epithelium *in vitro,* the low expression of this miRNA indicated damage of the proximal tubular epithelium after ischemia.

As shown in Figure 2, the levels of expression of miR-210, just like the miR-126, were heavily dependent on the availability of nutrients and oxygen, since the restriction of both decreased very significantly its expression with respect to the normality condition. The expression of miRNA was recovering in time of reoxygenation, where cells returned to dispose of oxygen and nutrients. The decrease of miR-210 indicated lack of nutrients and oxygen in the proximal tubular cells, being indicative, just like in the case of miR-126, of renal ischemia. On the other hand, and given that at 3 h of reoxygenation was recorded damage to the epithelium *in vitro,* the low expression of miR-210 indicated damage of the proximal tubular epithelium after ischemia.

As shown in Figure 3, the miR-101 increased its expression under hypoxia condition, compared to normoxia condition. The expression of this miRNA was normalizing again in reoxygenation where the availability of oxygen and nutrients was normalized again. The increase of expression of this miRNA in proximal cells during hipoxia condition was indicative of renal ischemia.

As shown in Figure 4, the miR-127 increased its expression under hipoxia condition compared to normoxia condition. The expression of this miRNA decreased significantly under reoxygenation, increasing again at 24 h of reoxygenation when the epithelial monolayer is recovering. Thus, the increase of expression of miR-127 in proximal cells during hipoxia condition was indicative of renal ischemia. The decrease of its early expression under reoxygenation indicated ischemic damage and its subsequent increase indicated endothelial recovery.

As shown in Figure 5, the expression of miR-101 was normalizing quickly under reoxygenation where the availability of oxygen and nutrients was normalized again, even though it stayed with higher levels of expression than under normoxia condition in this rat cells. The increase of expression of this miRNA in proximal cells during hipoxia condition was indicative of renal ischemia. Its new increase at 24 h of reoxygenation indicated recovery of the epithelial monolayer.

As shown in Figure 6, and just like in Hk-2 (human tubular) cells, miR-127 increased its expression under hipoxia condition in which the availability of nutrients and oxygen is restricted, compared to normoxia condition. The expression of this miRNA was normalizing quickly under reoxygenation where the oxygen and nutrients were available again. The increase of expression of this miRNA in proximal cells during hipoxia condition was indicative of renal ischemia.

As shown in Figure 7, and just like in proximal tubular cells, miR-101 increased its expression under hipoxia condition compared to normoxia condition. The expression of this miRNA was maintained high under reoxygenation where returned the availability of oxygen and nutrients were normalized again, and began to be normalized at 24 h of reoxygenation. The increase of expression of this miRNA in endothelial cells during hipoxia condition was indicative of renal ischemia. Its high expression under reoxygenation was associated with an endothelial activation (pro-inflammatory) status, which began to be normalized at 24 h.

As shown in Figure 8, just like in proximal tubular cells, miR-127 increased its expression under hipoxia condition in which the availability of nutrients and oxygen was restricted, compared to normoxia condition. The expression of this miRNA was maintained high under reoxygenation where oxygen and nutrients were available, and began to be normalized at 24 h of reoxygenation. The increase of expression of this miRNA in endothelial cells during hipoxia condition was indicative of renal ischemia. Its high expression under reoxygenation was associated with an endothelial activation (pro-inflammatory) status, which would begin to be normalized at 24 h.

As shown in Figure 9, and unlike of this miRNA, miRNA in proximal tubular cells, the miR-210 increased its expression discreetly under hipoxia condition, compared to normoxia condition. The expression of this miRNA was maintained high under and decreased and abruptly decreased its expression at 24 h of reoxygenation. The increase of expression of this miRNA in endothelial cells during hipoxia condition was indicative of renal ischemia. Just like the miR-127, and the miR-101, its expression under reoxygenation was associated with an endothelial activation (pro-inflammatory) status, which began to be normalized at 24 h.

As shown in Figure 10, the miR-126 increased its expression discreetly under hipoxia condition in which the availability of nutrients and oxygen was restricted, compared to normoxia condition, compared to normoxia condition. And it increased very significantly at 1 h of reoxygenation, where oxygen and nutrients were available. After that, the expression of this miRNA was normalized. The increase of expression of this miRNA in endothelial cells during hipoxia condition was indicative of renal ischemia. The increase so significant at 1 h of reoxygenation, it was associated unequivocally to a maximum endothelial activation (pro-inflammatory) status.

### EXAMPLE 2: Expression of miRNAs in patients that have been diagnosed with acute renal failure.

The study with samples of patients was carried out in a prospective way. After authorization by patients or their legal representatives through the pertinent informed consent and prior approval of the study by the Clinical Research Ethical Committee of our Hospital, samples of blood and urine and renal biopsies were extracted in the case of transplant in the patient groups described below.

### Samples of patients of kidney transplantation

Samples from 50 transplanted persons (patients with de novo kidney transplant and with different regimes of immunosuppression) were analyzed, organized into two groups:
I. 25 patients with immediate graft function
II. 25 patients with delayed graft function

Samples of blood and urine were taken on 1st, 7th, 15th, 30th days post renal transplant and in these days the expression of the miRNAs to be studied was determined by qRT-PCR.

In the case of graft non-function a biopsy was performed on the seventh day that was repeated every 7-10 days until the phase of ATN was resolved. In case of suspicion of rejection and after confirmation by biopsy, these patients were excluded.

In the case of transplanted patients, the following information was collected and determined:
- Recipient characteristics: age, sex, and time on dialysis.
- Donor characteristics: type of donor (encephalic death, asystolia), age, sex, vasoactive drugs need and last creatinine.
- Graft characteristics: times of hot, cold ischemia and of anastomosis. HLA compatibility and immunosuppression.
- Function of the graft at 2, 4 and 12 weeks.

Blood samples were collected in 8 ml VACUETTE tubes (z serum sep clot activator), that were centrifuged to 2500 rpm for 10 minutes.

The separated serum was collected by centrifugation and was aliquoted and stored in accordance with the criteria of the BioBank of the Ramon and Cajal Hospital (in anonymised tubes, with a unique code and at -80 °C).

Urine samples were collected in Standard vials of urine or extracted from the deposit of the probe, were centrifuged to 2500 rpm for 10 minutes to remove sediment and other residues, and were aliquoted and stored in accordance with the criteria of the BioBank of the Ramon and Cajal Hospital (in anonymised tubes, with a unique code and at -80 °C).

From all of them was requested its concession by the BioBank by means of concession agreements. A maximum of 500 microliters were requested since we have optimized the technology for amplifying miRNAs in samples of 100-200 microliters of both fluids, by quantitative PCR, for this purpose and after extraction of total RNA from samples of fluids (serum or urine) and titrates it, 50 nanograms of each one of them was used for the reaction of retrotranscription (RT) in 15 microliters. Special commercial primers were used for this step (stem loop primers). These primers were specific to each miRNA. After the RT, it was undertaken to the amplification reaction in a quantitative manner (qPCR). In this reaction that was carried out in a 10 microliters total volume, was used 1 microliter of the RT total reaction and specific primers for each miRNA and furthermore Taqman probe with fluorescence catchers. All reagents, both primers and reaction mixtures with enzymes and nucleotides for RT and PCR were utilized from Applied Biosystems.

Processing of the serum and urine samples of patients prior to the extraction of total RNA was as follows: an aliquot of 100-200 microliters of serum or urine, were digested with Proteinase K (0.65 milligrams/milliliter) incubating at 56 °C, 1 h. After that, a first extraction with phenol/chloroform (5:1) was carried out and the aqueous phase was processed using the High Pure miRNA isolation Kit (Roche), following the manufacturer's instructions.

### Samples of patients after cardiac surgery:

Samples from 50 patients were analyzed, organized into the following groups:
- IA: 10 adult patients operated on a scheduled way with extracorporeal circulation (ECC) and with low-risk for the ARF development, i.e. patients with a score from 0 to 2 in the Thakar5 system or from 0 to 1 in the SRI6 simplified.
- IB: 10 pediatric patients with congenital heart disease operated first-time with ECC.
- II: 15 adult patients operated on a scheduled way with ECC, with altered basal renal function and scores >5 in the Thakar5 system or ≥ 3 in the SRI6.
- III: 15 adult patients operated on a scheduled way with ECC, with normal basal renal function and with the same scores as in the previous section.

For each patient determinations of the miRNAs cited in the following moments were made:
- Basal preoperative
- At 2 h on admittance at ICU
- After 24 h, 48 h and 72 h of surgery
- On day 7 (optional for groups IA and IB)

As shown in Figure 11, the expression of the miR-127 was much diminished on the healthy control. The decrease in the expression of this miRNA in the serum of patients was indicator of renal ischemic damage or ARF. These data correlate with the data shown in the figure 18.

As shown in Figure 12, and in correlation with the serum decrease, the expression of the miR-127 increased significantly on the healthy control. The decrease in the expression of this miRNA in the serum of patients and its corresponding urine increase was indicator of early diagnosis of renal ischemic damage or ARF.

As shown in Figure 13, the levels of serum miR-126 decreased significantly its expression at the time of onset of ischemic renal damage and increased a lot its expression at 24 h, to be subsequently normalized. This indicated that the decrease in the expression of this miRNA in the serum of patients was indicator of early diagnosis of renal ischemic damage or ARF. Its increase at 24 h indicated an endothelial activation after ischemia associated with a subsequent inflammatory reaction, as discussed in the *in vitro* model. These data correlate with the data shown in the figure 19.

As shown in Figure 14, the serum miR-210 increased significantly its expression at the time of onset of ischemic renal damage; it is maintained in the first 24 h, in order to be subsequently normalized. These data indicated that the increase in the expression of this miRNA in the serum of patients was an early diagnostic marker of renal ischemic damage or ARF.

As shown in the Figure 15, the urinary miR-210 increased significantly its expression at 48 h, to be subsequently normalized. Taking into account that this miRNA increased its expression in HK-2 cells at times in which an epithelial recovery in the experimental model began to be observed, this indicated that the increase in the expression of this miRNA at 48 h in urine of patients was indicative of the onset of recovery after renal ischemic damage. These data correlate with the data shown in the figure 20.

As shown in Figure 16, the expression of the miR-101 increased significantly early with regard to healthy control, at the time of admittance, and a significant expression with regard to the healthy subject in the time of progress was no longer detected. This indicated that the increase in the expression of this miRNA in serum of patients was an early diagnostic marker of renal ischemic damage. These data correlate with the data shown in the figure 17.

## Claims

1. Method for the diagnosis and/or prognosis of acute renal damage comprising analyzing a sample of a patient, to determinate the level of expression of at least un microRNA selected from miR-127, miR-126, miR-210 and miR-101 and compare said level of expression with a control value, where the alteration of said level is indicative of acute renal damage.

2. Method for the diagnosis and/or prognosis of acute renal damage according to claim 1, where the sample to be analyzed is selected from blood, serum or urine.

3. Method for the diagnosis and/or prognosis of acute renal damage according to any of claims 1-2, where the decrease of the level of expression of serum miR-127 with regard to the control value is indicative of acute renal damage.

4. Method for the diagnosis and/or prognosis of acute renal damage according to any of claims 1-2, where the increase of the level of expression of urinary miR-127 with regard to the control value is indicative of acute renal damage.

5. Method for the diagnosis and/or prognosis of acute renal damage according to any of claims 1-2, where the decrease of the level of expression of serum miR-126 with regard to the control value is indicative of acute renal damage.

6. Method for the diagnosis and/or prognosis of acute renal damage according to any of claims 1-2, where the increase of the level of expression of serum miR-210 with regard to the control value is indicative of acute renal damage.

7. Method for the diagnosis and/or prognosis of acute renal damage according to any of claims 1-2, where the decrease of level of expression of urinary miR-210 with regard to the control value is indicative of acute renal damage.

8. Method for the diagnosis and/or prognosis of acute renal damage according to any of claims 1-2, where the increase of level of expression of serum miR-101 with regard to the control value is indicative of acute renal damage.

9. Method for the diagnosis and/or prognosis of acute renal damage according to any of the preceding claims, where the expression of microRNA is determined by PCR.

10. Method for the diagnosis and/or prognosis of acute renal damage according to any of the preceding claims, where the expression of microRNA is determined by quantitative PCR.

11. Method according to any of claims 1-8, where the level of expression of microRNA is determined by ARN microarrays.

12. Kit for the diagnosis and/or prognosis of acute renal damage comprising the probes and primers needed for carrying out the method according to any of claims 1-11.
